# EUROPEAN PATENT SPECIFICATION

(11) **EP 3 439 539 B1**
(45) Date of publication and mention of the grant of the patent: **11.09.2019**
(21) Application number: 17712523.4
(22) Date of filing: 27.03.2017
(51) Int. Cl.: A61B 5/00, A45D 2/00, A61B 5/04, G01N 33/36, G01N 27/60

(54) **FIBER QUALITY SENSOR**
FASERQUALITÄTSSENSOR
CAPTEUR DE QUALITÉ DE FIBRES

(30) Priority: 08.04.2016 EP 16164516
(43) Date of publication of application: 13.02.2019
(73) Proprietor: Koninklijke Philips N.V., 5656 AE Eindhoven (NL)
(72) Inventor: GERHARDT, Lutz, Christian, 5656 AE Eindhoven (NL); ASVADI, Sima, 5656 AE Eindhoven (NL); VAN LIEROP, Michael, Maria, Johannes, 5656 AE Eindhoven (NL); JOYE, Neil, Francis, 5656 AE Eindhoven (NL); JOHNSON, Mark, Thomas, 5656 AE Eindhoven (NL); OUWERKERK, Martin, 5656 AE Eindhoven (NL)
(74) Representative: Steenbeek, Leonardus Johannes
(86) International application number: PCT/EP2017/057144
(87) International publication number: WO 2017/174381

(56) References cited:
- EP-A1- 2 394 944
- WO-A1-2014/020119
- JP-A- S61 221 641
- US-B1- 6 650 959
- ANTHONY C LUNN ET AL: "The electrostatic properties of human hair", JOURNAL OF THE SOCIETY COSMETIC CHEMISTS, SOCIETY OF COSMETIC CHEMISTS, US , vol. 28, no. 9 1 September 1977 (1977-09-01), pages 549-569, XP002714504, ISSN: 0037-9832 Retrieved from the Internet: URL:http://journal.scconline.org/pdf/cc197 7/cc028n09/p00549-p00569.pdf [retrieved on 2013-10-08]

## Description

### FIELD OF THE INVENTION

The invention relates to a fiber quality sensor such as a hair damage sensor, and to a hair care device comprising such a hair damage sensor.

### BACKGROUND OF THE INVENTION

The chapter 7 "Surface Potential Studies of Human Hair Using Kelvin Probe Microscopy", by Bhushan in: Biophysics of Human Hair: Structural, Nanomechanical, and Nanotribological Studies, pp. 153-169 (2010), discloses that virgin hair has a better charge mobility and can therefore dissipate charge more readily than chemically damaged hair.

US 6,518,765 describes using an array of triboelectric sensors for testing electrostatic properties of a remote environment. A method of determining the triboelectric properties of a material comprises selecting a plurality of insulators; simultaneously rubbing the plurality of insulators against the material; measuring a change in a magnitude and polarity of an electrical charge on each of a plurality of the insulators over time; and determining a triboelectric property of the material in response to results from said measuring. The insulating materials may be selected so their triboelectric properties cover a desired range.

US 2003/226397 describes a directional coupler sensor for measuring the moisture content of a substrate, such as hair. The sensor incorporates a high frequency directional coupler having a pair of generally parallel plates defining a coupling gap therebetween. A high frequency signal generator generates an electromagnetic field across the gap with the substrate placed across the coupling gap. The coupled power relates to the moisture content of the substrate. A pressure sensor is provided to ensure that the desired compactness of the substrate across the coupling gap is achieved to obtain accurate, reliable and consistent results.

US 2016/0028327 describes a method of producing a triboelectric generator element comprising a material based on rough dielectric polymer intended, in order to create electrical charges, to be placed in contact with another material having different triboelectric properties to those of the dielectric polymer material, the method including forming on a support a layer based on a material formed of a given dielectric polymer.

US 2016/0011233 describes a sensor for measuring static charge of fibers, comprising: a sensor handle which is insulated; a metal sensor head connecting to the sensor handle; an electrometer and a capacitor, both inside of the insulated sensor handle (whereby the isolated handle is not in direct contact with the fiber and does not result in an electrical signal); and a display on the handle, and wherein the static charge generated during a contact between the fiber and the sensor head is transferred from the sensor head to the capacitor, measured by the electrometer connected to the capacitor, and shown on the display. The sensor head is preferably in the shape of a brush or comb, and the static charge is generated during combing. Measuring electrostatics of fibers, especially when combing fibers is one of common ways to assess keratinaceous fiber conditions. Generally speaking, more electrostatics on fibers cause more fly-away of fibers. More damaged and/or curled keratinaceous fibers may cause more electrostatic charging when combing because of more friction and/or detangling between fibers when combing.

US 6504375 describes an electrostatic voltmeter modulator for measuring an electrostatic field between the electrostatic voltmeter modulator and a surface includes a shield, a sensing electrode, and a layer disposed between the shield and sensing electrode, Document "The electrostatic properties of human hair" by Anthony C. Lunn and Robert E Evans in Journal Of The Society Cosmetic Chemists, 28, 549-569 from September 1977 discloses an apparatus for measuring the magnitude of charge generated by combing human hair, the mobility of charge and the distribution of charge along the length of hair fibers.

### SUMMARY OF THE INVENTION

It is, inter alia, an object of the invention to provide a fiber quality sensor. The invention is defined by the independent claims. Advantageous embodiments are defined in the dependent claims.

Embodiments of the invention provide a hair based triboelectric sensor device with self-generating sensor signal to detect hair health or damage, and in particular a sensor device that measures and analyses the signal shape, magnitude of hair surface potential and rate of hair surface discharging (charge retention/dissipation) during a hair treatment with a hair care device, such as a straightener. The sensor technology is based on the analysis of electrostatic and/or triboelectric surface charging characteristics of hair fibers. The hair based sensing technology with self-generating signal offers potential for both a stand-alone consumer and/or professional device and an integrated module into existing hair styling devices (e.g. hair straightener, styler). The hair based sensor is based on but not limited to a sliding triboelectric generator being used as a sensor for measuring a charge (voltage or surface potential change) which is dependent on the surface property (topography, chemistry) of hair. The sensor principle makes use of the triboelectric effect, in-plane charge separation and electrostatic induction. The rubbing of a hair against a counter-surface (e.g. of a care device) causes an electrical charge build-up and charge induction in a specific sensor electrode array, made out of a conductive and dielectric electrode allows to monitor the time-dependent and spatial, macroscopic discharge behavior. The charge leaks away through the hair and/or sensor specific induction electrode materials/configurations. The rate of this process depends on the structural (integrity of cuticles, number of cuticle layers removed) and physicochemical surface properties of the hair fiber (presence of lipids, moisture level). By comparing the voltage signature with reference shapes and analyzing the signal in the time and frequency domain, the level of hair damaged can be assessed and quantified (if a calibration curve is available), or a baseline measurement done.

These and other aspects of the invention will be apparent from and elucidated with reference to the embodiments described hereinafter.

### BRIEF DESCRIPTION OF THE DRAWINGS

Fig. 1 illustrates the principle of the invention;
Fig. 2 shows a first embodiment of a hair damage sensor in accordance with the invention;
Fig. 3 shows a (multiple) interdigitated hybrid induction electrode pair embodiment for hair damage sensing/detection according to the invention;
Figs. 4A, 4B and 4C show different electrode arrangements on hair straightener plate to compensate for angular deviation and hair length difference at hair tips; and
Figs. 5, 6A and 6B show examples of electrode configurations for use in a fiber quality sensor according to the invention.

### DESCRIPTION OF EMBODIMENTS

Embodiments of the invention are defined by the appended claims. In particular, embodiments provide a sensor device that measures and analyses the signal shape, magnitude of hair surface potential and rate of hair surface discharging (charge retention/dissipation) during a hair treatment with a hair care device, such as a straightener. The sensor is based on a hair based sliding triboelectric generators for generating charge in response to movement of the hair care device over the hair; and this hair based triboelectric generator being used as a sensor for measuring a parameter which is dependent on the surface property (topography, chemistry), the sensor signal being the charge (voltage) generated by the triboelectric generator. The sensor principle makes use of the triboelectric effect, in-plane charge separation and electrostatic induction. The rubbing of a hair against a counter-surface (e.g. of a hair care device) causes an electrical charge build-up and charge induction in a specific sensor electrode array, made out of a conductive and dielectric electrode allows to monitor the time-dependent and spatial, macroscopic discharge behavior. The charge leaks away through the hair and/or sensor specific induction electrode materials/configurations. The rate of this process depends on the structural (integrity of cuticles, number of cuticle layers removed) and physicochemical surface properties of the hair fiber (presence of lipids, moisture level). By comparing the voltage signature with reference shapes and analyzing the signal in the time and frequency domain, the level of hair damaged can be assessed and quantified (if a calibration curve is available), or a baseline measurement done.

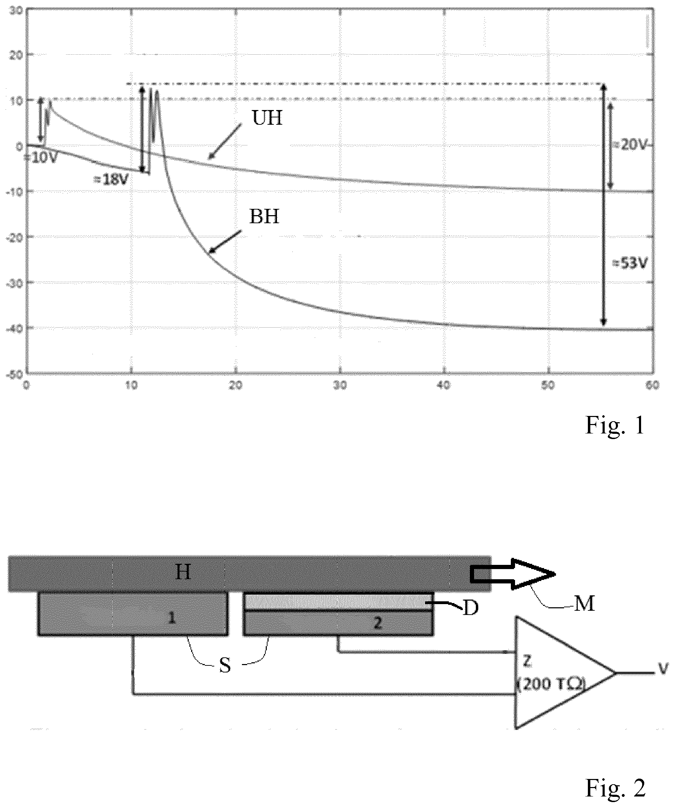

Fig. 1 shows triboelectric hair surface potential changes in Volt of untreated hair UH and bleached hair BH (using commercially available chemical bleaching products) versus time in seconds, measured during a one-directional stroke of blond hair over a specific electrode configuration subject of this invention. The curve BH shows that bleached hair can build up more surface charges (higher peak-peak voltage Vpp: 18 V vs. 10 V) but charges do leak away faster and to a greater extent (saturation voltage: -40 V vs. -10 V) compared to unbleached hair UH. The curve UH for unbleached hair shows an increase in potential (2^{nd} peak is larger than 1^{st} peak), whereas the charge leakage is faster for bleached hair BH indicated by the fact that the 2^{nd} peak is lower and/or as high as the 1^{st} peak. The minimum is also lower for bleached hair BH than for unbleached hair UH indicating greater leakage rate. While the step amplitude ratios of the leading edge of two successive peaks are similar (12 V / 18 V for bleached hair BH, 5 V / 8 V for unbleached hair UH) and presumably related to the (geometric) properties of the electrodes, the absolute peak height ratio of two successive peaks gives information on the leakage rate and charge retention properties of hair, and hair condition (e.g. presence of lipids, moisture level). Absolute peak height ratios <1 indicate charge leakage dominating charge build up in time (for bleached hair BH: 11.5 V / 12 V), ratios >1 indicate charge build up dominating charge leakage in time (for unbleached hair UH: 10 V / 8 V). It has to be noticed that the height of the 1^{st} and 2^{nd} peaks, and therefore the absolute peak height ratio, also depends on the triboelectric sensor configuration and speed of the stroke.

To be able to use this invention in a handheld device, an electrode pair or grating structure - together with the hair forming a sliding triboelectric generator - is integrated in the hair styling device and responds to the surface charge build-up by means of triboelectric charging and electrostatic induction. Suitable electronics to capture the signal/charge build up is an amplifier with large input resistance (typically > 10¹² Ω).

In a first embodiment of this invention, a specific hybrid electrode design made out of conductive and dielectric electrodes allows to monitor the time-dependent and spatial, macroscopic discharge behavior. The conductive part is made of a conductive material such as copper (Cu), aluminum (Al) or a transparent conductor such as indium tin oxide (ITO), poly(3,4-ethylenedioxythiophene) polystyrene sulfonate (PEDOT-PSS), or graphene. The dielectric part is made of a dielectric material such as polyester (PET) or fluorinated ethylene propylene (FEP). This invention is not limited to the listed materials. Combinations of many other suitable materials could also be used; a person skilled in the art knows how to combine them correctly based on their tribo-electric properties. An in-plane induction electrode pair or multiple interdigitated electrodes made out of one metallized dielectric (metal is on the back side) and one conductive material enable to measure large flat hair bundles and assess the hair damage state based on controlled discharge through the conductive electrode(s). Hair rubbed over these electrode arrays result in multiple in-plane charge separation and induction events but also 'forced' discharge events due to conductive metal electrode allowing one to study the dynamic discharge behavior of hair.

Fig. 2 shows a first embodiment of a triboelectric fiber quality sensor as a hair damage sensor in accordance with the present invention with alternating conductive and dielectric electrode segments. M indicates a unidirectional movement of the sensor S along the hair H. The sensor S includes two electrodes 1 and 2, the second electrode 2 being covered by a dielectric D. The output voltages of the electrodes 1 and 2 are applied to an amplifier having a very high input impedance Z of about 200 TΩ. The very large input impedance Z of the amplifier insures that the charges present on the electrodes 1, 2 leak very slowly into the amplifier. When being rubbed over such a dielectric-conductive electrode pair or alternating array of dielectric-conductive electrodes, a specific voltage potential across the electrode pair is generated. This voltage potential is due to the triboelectric charges and electrostatic induction being generated at the hair/sensor interface. Moreover, charge leakage from the hair to the conductive electrode 1 (e.g. copper electrode) has also an influence on the measured voltage. Thus, the voltage potential measured across the electrode pair (partially) depends on the hair surface topography/chemistry and therefore allows to characterize the health state of hair.

If the unique signal signature deviates, this allows a statement on the charging state/characteristics and surface condition of the hair (see embodiment 4).

All the herein described in-plane electrode pairs/arrays can, for example, be integrated in a plate of a hair straightener.

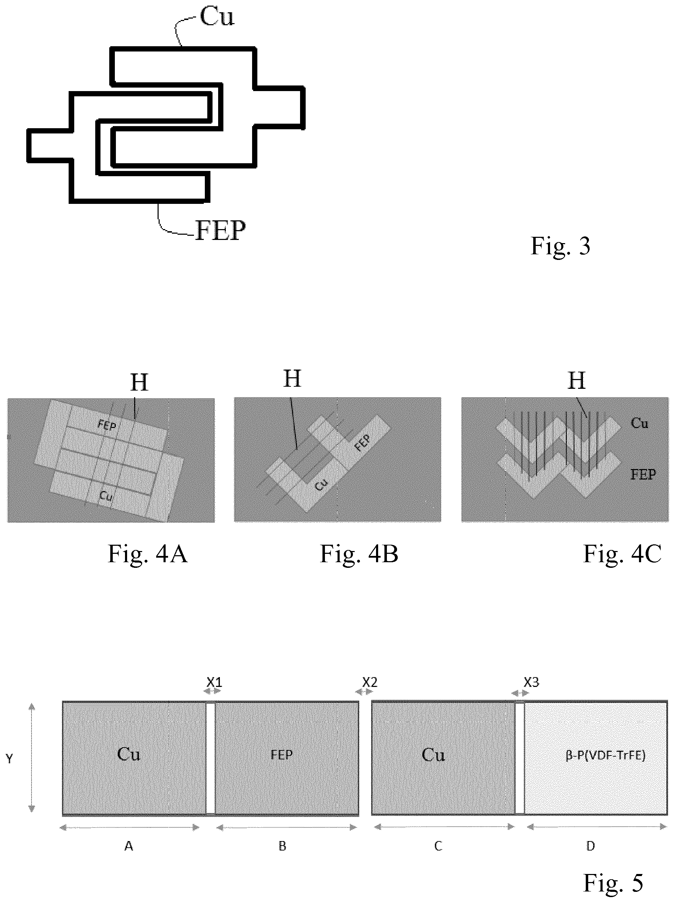

Fig. 3 shows a (multiple) interdigitated hybrid induction electrode pair embodiment for hair damage sensing/detection according to the invention. Dielectric = FEP; conductor = copper Cu. As in Fig. 2, the FEP electrode has a copper backing.

In a second embodiment of this invention, various geometries of hybrid electrodes are used to compensate for angular deviation and avoid signal noise at the hair tip during use of a hair care device. These geometries are aimed to improve the sensor signal. To fully exploit the sensor signal, in the ideal case the hair should be rubbed substantially perpendicularly relative to the orientation of the electrodes pairs. In many hair care applications it may be difficult to maintain or control such a substantially perpendicular orientation, or the device is used under a certain angle relative to the length of the hair bundle or root-tip direction. Therefore, specific electrode design configurations are needed making straightening robust to DC noise burst which would be generated instead of an alternating voltage signal during sliding at the hair tip. By using specific arrangements and modifications of the above described hybrid electrode (embodiment 1), such a noise burst can be avoided and clear signals extracted. For compensating angular deviation from a perfect 90 degree angle or angular misalignment, and improve the sensor signal (signal-to-noise ratio), dedicated geometries can include the following electrode geometries or combinations thereof:
- Slanted/tilted electrodes
- V-shaped electrodes
- Zig-zag shaped electrodes
- Meander-like electrodes
- Staggered electrodes
- Alternating electrodes with asymmetric width of electrode fingers (e.g. width of conductive electrode twice the width of dielectric electrode to improve discharge events).

Figs. 4A, 4B and 4C show different electrode arrangements on hair straightener plate to compensate for angular deviation and hair length difference at hair tips. Fig. 4A shows slanted / tilted electrodes, Fig. 4B shows V-shaped electrodes, and Fig. 4C shows zig-zag shaped electrodes as can be provided on a straightener plate of a hair care device.

In a third embodiment, similar to embodiments 1 and 2, an in-plane electrode configuration of alternating electrodes materials is used. In this embodiment there is one additional material, and thus in total three types of materials, viz. (1) an electrically conductive material, such as a metal like copper (Cu), (2) a material which is lower than the chosen electrically conductive material in the triboelectric series compared to hair, for example fluorinated ethylene propylene (FEP) or Teflon types, and (3) a third material which is ranked above the chosen electrically conductive material (e.g. copper Cu) in the triboelectric series. Ideally, this third material should also be ranked above hair, e.g. polyurethane or negatively charged P-P(VDF-TrFE). See e.g. https://www.trifield.com/content/tribo-electric-series/, http://www.regentsprep.org/regents/physics/phys03/atribo/default.htm, or http://www.rfcafe.com/references/electrical/triboelectric-series.htm for a table listing various materials according to their respective affinities for negative charge.

The electrode configuration shown in Fig. 5 is used as an example. Similar to the previous embodiments, the voltage potential between electrodes A and B is monitored. Additionally, the voltage potential between electrodes C and D is also monitored. The configurations shown in Fig. 5 do not make the output "insensitive" to the brushing direction. To do this, one would need to "mirror" the configurations e.g. ABCDCDAB or even ABBACDDC. As in the previous embodiments, the FEP and β- Poly(vinylidene fluoride-trifluoroethylene) (in short: β-P(VDF-TrFE)) electrodes are metalized at the back. Symbols A until D; XI until X3, and Y denote the dimensions of the electrodes. These dimensions can be varied to get an optimal signal.

When hair strands are rubbed over the surface or vice versa, starting on the nonconductive segments and moving into the conductive segments, two electrical signals are generated. The usage of two triboelectric charge generating materials, one higher and one lower in the triboelectric series compared to copper, results in two electrical signals which are mainly in opposite direction of each other (respectively positively and negatively).

This additional signal and the ratio between these signals can be used to gain additional spatial information on the surface chemical state of hair (e.g. relative presence of polar and non-polar groups varying over the full hair length (from root to tip) or the individual cuticle length (typical 3-5 µm, i.e. suggested width of 3^{rd} electrode material is 1 to 3 µm). To compensate for or nullify the misalignment (un-parallelism of active tribo areas in respect to cuticle direction), the electrode should have multiple small segments of e.g. 10 by 10 µm in length and/or width, preferably lengths and widths in the order of 1 to 5 µm. These dimensions of the active tribo area are not limited to the third material and can be used for one or more of the other used materials. The relatively uniform potential on the cuticle when measured far away from the cell borders increases close to the edge of the cuticle cells, indicating that these regions are more polar due to protein exposure.

Furthermore it can be used to distinguish between combing direction, from tip to root or from root to tip, as this difference in combing direction results in different electrical output signals.

By changing the electrode dimensions denoted in Fig. 5 by symbols A until D, X1-X3, and Y, the electrical output signal can be adjusted. For example, the area of the FEP and β-P(VDF-TrFE) covered electrodes can be increased to increase the charging surface.

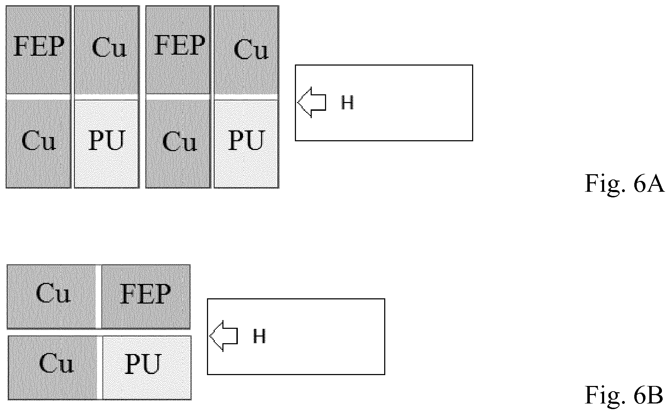

Furthermore multiple segments and combinations of these segments can used. For example, the configuration of Fig. 5 can be repeated horizontally and/or vertically. Figs. 6A and 6B show some other examples, with electrodes made of copper (Cu), fluorinated ethylene propylene (FEP) and polyurethane (PU), and these configurations too can be repeated horizontally and/or vertically. Fig. 6A shows alternating conductive and charge generating surfaces, and Fig. 6B shows both charge generating surfaces in front followed by the conductive segments in relation to the movement of the sensor over hair H.

In addition, the analysis of a modulated charge dissipating signal (which is dependent on the hair surface topography/chemistry properties and the electrode design such as width of electrode segments) allows to conclude on the hair damage state. As mentioned in relation to embodiment 2, the electrode layout dictates the shape of the generated triboelectric voltage. The signal is modulated and results in a distinctive pattern of repeated pulses. Damage detection of hair by signal analysis of distinctive triboelectric surface potential patterns is possible by means of a suitably programmed microprocessor through:
- Comparison of triboelectric signal with deviation from standard signature of undamaged or untreated/unbleached hair.
- Characteristic shape of modulated signal in time domain or spatial domain: Relating time to displacement/movement during rubbing over electrode segments.
- Charge exchange, retention/dissipation rate (1^{st} derivative) of triboelectric surface potential signals: time derivative.
- Frequency spectra (harmonics, ratio of harmonics, frequency range).

Embodiments of the invention thus provide a new sensor technology with self-generating signal based on triboelectric effect and electrostatic charge induction. A specific electrode configuration is provided, with an array of two in-plane electrodes with alternating conductive and dielectric coated segments: alternating conductive (charge dissipation electrode) and dielectric covered induction electrodes. A main advantage of this feature is that it allows monitoring charge build up and discharge behavior/events of a fiber like hair. This configuration fundamentally differs from a conventional triboelectric generator in that in such a conventional triboelectric generator a conductive discharge electrode would not be placed directly next to a dielectric electrode as this would negatively affect power generation/sensor signal magnitude as charges leak away. The controlled or forced sequential discharge monitoring of hair allows discriminating healthy from damaged hair based on charge leakage/dissipation behavior as shown in Fig. 1. Such a controlled deliberate charge leakage is desired in order to be able to characterize healthy and damaged hair based on typical discharge signals. Minimum additional electronics and system architectural changes are needed for implementation of sensor in hair straightener. A device with an in-built triboelectric sensor system for hair health sensing can be realized: no external power supply needed to power the sensor. Hair is part of a sensor system forming a triboelectric sensing device with self-generating signal.

An embodiment provides a in-plane electrode array with alternating dielectric (polymer insulator) coated and conductive (metallic) electrode segments. The triboelectric charge generated during movement of a third material (fiber, hair) over the electrodes (or vice versa) results in a surface potential difference measured between both electrodes. Other types of triboelectric generators are alternatively possible, e.g. a vertical contact separation (tapping mode) generator. Alternatively, by just consecutively tapping the fibers a unique charge-discharge signature can be obtained. Any form of contact electrification or triboelectric charging that produces an electrical signal that can be measured can be employed in the present invention. The conductive electrode induces controlled repeated discharge events during rubbing and functions mainly as a charge dissipation electrode. The combination of triboelectric / electrostatic charging and controlled discharging during the rubbing produces a unique signature of the fiber that can be used by a microprocessor to determine surface electrochemical and topographical properties (such as damage, surface integrity, electro-chemical surface state).

An embodiment provides a sensor that allows to measure both the build-up and discharge of electrostatic charges simultaneously during rubbing using specific alternating dielectric and conductive electrode segment configurations, whereafter the generated charges are converted into a voltage using a high-ohmic electrometer.

An embodiment provides a fiber sensor system comprising a pair or an array of alternating conductive and dielectric coated segments (insulators), a third fiber-like material and an amplifier connected to the pair of alternating electrodes and having an input impedance in the range 1 - 200 TOhm. The triboelectric charge generated during movement of the third material over the electrodes (or vice versa) results in a surface potential difference measured between both electrodes. The conductive electrode induces controlled repeated discharge events during rubbing and functions mainly as a charge dissipation electrode to characterize the electrochemical and topographical surface state of the fiber.

An embodiment of the invention comprises an electrode array with alternating dielectric (insulator) coated and conductive (metallic) electrode segments, differential measurement of surface potential changes, special sensor electronics with input resistance being at least 1 TOhm, and measurement of combined electrostatic charge and discharge (rate) behavior to provide a unique triboelectric fiber signature.

Possible applications include:
- Sensor for sensing surface damage (e.g. coating imperfections/doped surfaces) based on triboelectric charges and electrostatic discharge phenomena.
- Hair based triboelectric sensor device with self-generating sensor signal to detect hair damage.
- Hair is part of a sensor system forming a triboelectric sensing device.
- Use triboelectric surface potential (tribo-voltages generated between the hair surface and the counter-surface of a hair care device) as an indicator/measure for hair health/damage.
- Methods to characterize macroscopic surface charging mechanisms of dielectric or badly conducting coatings: signal analysis in time and frequency domain.
- Methods to discriminate between undamaged and damaged hair: signal analysis in time and frequency domain.
- Handheld apparatus suitable for sensing surface damage (e.g. coating imperfections/doped surfaces) based on triboelectric charges and electrostatic discharge phenomena.
- Device inherent triboelectric sensor with self-generating sensor signal for monitoring hair health/damage.
- Handheld apparatus for triboelectric sensing of hair health and pre-warning for over-treatment.

The invention is advantageously used in a hair styling device, comprising a fiber quality sensor S according to the invention, and a microprocessor coupled to receive output signals from the fiber quality sensor. The hair styling device could, for example, provide an indication (e.g. optical or acoustic alarm) to the user that he/she should stop the treatment in order to avoid overheating. Settings (e.g. temperature) could also be adapted.

The invention can be applied not only in hair styling applications, but also in textile (fiber) applications, e.g. monitoring quality and durability of textile finishing during production, or in garment care, e.g. monitoring quality and durability of textiles during use or after laundering.

It should be noted that the above-mentioned embodiments illustrate rather than limit the invention, and that those skilled in the art will be able to design many alternative embodiments without departing from the scope of the appended claims. In the claims, any items placed between parentheses are just reference signs referring to examples in the drawings that shall not be construed as limiting the claim. In particular, the scope of the claims is not limited by materials matching the reference signs, so that instead of copper (Cu) any suitable conductive materials may be used, while instead of FEP and PU other suitable dielectric materials may be used. The word "comprising" does not exclude the presence of elements or steps other than those listed in a claim. The word "a" or "an" preceding an element does not exclude the presence of a plurality of such elements. The notion "a pair" thus includes a plurality of pairs, and the notion "a pair of mutually adjacent electrodes" includes interdigitated electrode arrays or multiple interdigitated electrodes.

## Claims

1. A triboelectric fiber quality sensor (S) for sensing a quality of a fiber (H), the fiber quality sensor (S) comprising a first in-plane pair of mutually adjacent electrodes arranged for contacting the fiber (H) to generate a first voltage over the electrodes, the first voltage being indicative of the quality of the fiber (H), the first pair of electrodes including:
a first conductive electrode (1, Cu), and
a first dielectric electrode (D, 2) having a first dielectric material (D, FEP) with a conductive backing (2).

2. A triboelectric fiber quality sensor (S) as claimed in claim 1, the triboelectric fiber quality sensor further comprising a second in-plane pair of mutually adjacent electrodes arranged for contacting the fiber (H) to generate a second voltage over the electrodes, the second voltage being indicative of the quality of the fiber (H), the second pair of electrodes including:
a second conductive electrode (1, Cu), and
a second dielectric electrode (D, 2) having a second dielectric material (PU) with a conductive backing, wherein in a triboelectric series of materials the material of the conductive electrode (Cu) is arranged between the first (FEP) and second (PU) dielectric materials.

3. A triboelectric fiber quality sensor (S) as claimed in claim 2, comprising horizontally or vertically alternating first and second in-plane pairs of electrodes.

4. A triboelectric fiber quality sensor (S) as claimed in any of the preceding claims, wherein the first and/or second in-plane pairs of electrodes are tilted, slanted and/or zigzag-shaped, or wherein in-plane pairs of electrodes are arranged in a repeated interdigitated pattern such as an interdigitated grating structure.

5. A triboelectric fiber quality sensor (S) as claimed in any of the preceding claims, for the or each in-plane pair of electrodes further comprising an amplifier coupled to the pair of electrodes and having an input impedance exceeding 1 TΩ, and preferably being 200 TΩ.

6. A hair styling device, comprising:
a triboelectric fiber quality sensor (S) as claimed in any of the preceding claims.

7. A hair styling device as claimed in claim 6, further comprising a microprocessor for analyzing the first voltage signal.

8. A hair styling device as claimed in claim 7, further comprising an indicator for, in response to an output of the microprocessor, providing an indication to a user that the user should stop a treatment in order to avoid overheating.

9. A hair styling device as claimed in claim 7 or 8, wherein the hair styling device is arranged for modifying a temperature setting in response to an output of the microprocessor.

## Patentansprüche

1. Triboelektrischer Faserqualitätssensor (S) zum Erfassen einer Faserqualität (H), wobei der Faserqualitätssensor (S) ein erstes ebenengleiches Paar von einander benachbarten Elektroden umfasst, das zum Kontaktieren der Faser (H) angeordnet ist, um eine erste Spannung über den Elektroden zu erzeugen, wobei die erste Spannung die Faserqualität (H) anzeigt, wobei das erste Paar von Elektroden aufweist:
eine erste leitende Elektrode (1, Cu) und
eine erste dielektrische Elektrode (D, 2), die ein erstes dielektrisches Material (D, FEP) mit einer leitfähigen Unterschicht (2) aufweist.

2. Triboelektrischer Faserqualitätssensor (S) nach Anspruch 1, wobei der triboelektrische Faserqualitätssensor weiter ein zweites ebenengleiches Paar von einander benachbarten Elektroden umfasst, das zum Kontaktieren der Faser (H) angeordnet ist, um eine zweite Spannung über den Elektroden zu erzeugen, wobei die zweite Spannung die Faserqualität (H) anzeigt, wobei das zweite Paar von Elektroden aufweist:
eine zweite leitende Elektrode (1, Cu) und
eine zweite dielektrische Elektrode (D, 2), die ein zweites dielektrischen Material (PU) mit einer leitfähigen Unterschicht aufweist, wobei bei einer triboelektrischen Reihe von Materialien das Material der leitenden Elektrode (Cu) zwischen dem ersten (FEP) und dem zweiten (PU) dielektrischen Material angeordnet ist.

3. Triboelektrischer Faserqualitätssensor (S) nach Anspruch 2, umfassend horizontal oder vertikal abwechselnde erste und zweite ebenengleiche Paare von Elektroden.

4. Triboelektrischer Faserqualitätssensor (S) nach einem der vorstehenden Ansprüche, wobei das erste und/oder zweite ebenengleiche Paar von Elektroden gekippt, abgeschrägt und/oder zickzackförmig sind oder wobei die ebenengleichen Paare von Elektroden in einem wiederholten ineinandergreifenden Muster, wie einer ineinandergreifenden Gitterstruktur, angeordnet sind.

5. Triboelektrischer Faserqualitätssensor (S) nach einem der vorstehenden Ansprüche, wobei das oder jedes ebenengleiche Paar von Elektroden weiter einen Verstärker umfasst, der mit dem Paar von Elektroden gekoppelt ist und eine Eingangsimpedanz aufweist, die 1 TΩ überschreitet und vorzugsweise 200 TΩ beträgt.

6. Haarfrisiervorrichtung, umfassend:
einen triboelektrischen Faserqualitätssensor (S) nach einem der vorstehenden Ansprüche.

7. Haarfrisiervorrichtung nach Anspruch 6, weiter umfassend einen Mikroprozessor zum Analysieren des ersten Spannungssignals.

8. Haarfrisiervorrichtung nach Anspruch 7, weiter umfassend einen Indikator zum Bereitstellen, als Reaktion auf eine Ausgabe des Mikroprozessors, einer Anzeige an einen Benutzer, dass der Benutzer eine Behandlung anhalten sollte, um eine Überhitzung zu vermeiden.

9. Haarfrisiervorrichtung nach Anspruch 7 oder 8, wobei die Haarfrisiervorrichtung zum Modifizieren einer Temperatureinstellung als Reaktion auf eine Ausgabe des Mikroprozessors ausgelegt ist.

## Revendications

1. Capteur de qualité de fibre triboélectrique (S) destiné à détecter une qualité d'une fibre (H), le capteur de qualité de fibre (S) comprenant une première paire d'électrodes dans le plan mutuellement adjacentes agencées pour entrer en contact avec la fibre (H) pour générer une première tension sur les électrodes, la première tension étant indicative de la qualité de la fibre (H), la première paire d'électrodes comprenant :
une première électrode conductrice (1, Cu), et
une première électrode diélectrique (D, 2) ayant un premier matériau diélectrique (D, FEP) avec un support conducteur (2).

2. Capteur de qualité de fibre triboélectrique (S) selon la revendication 1, le capteur de qualité de fibre triboélectrique comprenant en outre une seconde paire d'électrodes mutuellement adjacentes dans le plan agencées pour entrer en contact avec la fibre (H) pour générer une seconde tension sur les électrodes, la seconde tension étant indicative de la qualité de la fibre (H), la seconde paire d'électrodes comprenant :
une seconde électrode conductrice (1, Cu), et
une seconde électrode diélectrique (D, 2) ayant un second matériau diélectrique (PU) avec un support conducteur, dans lequel dans une série triboélectrique de matériaux, le matériau de l'électrode conductrice (Cu) est agencé entre les premier (FEP) et second (PU) matériaux diélectriques.

3. Capteur de qualité de fibre triboélectrique (S) selon la revendication 2, comprenant des première et seconde paires d'électrodes dans le plan alternées horizontalement ou verticalement.

4. Capteur de qualité de fibre triboélectrique (S) selon l'une quelconque des revendications précédentes, dans lequel les première et/ou seconde paires d'électrodes dans le plan sont inclinées, penchées et/ou en forme de zigzag, ou dans lequel des paires d'électrodes dans le plan sont agencées selon un motif entrecroisé répété, tel qu'une structure de grille entrecroisée.

5. Capteur de qualité de fibre triboélectrique (S) selon l'une quelconque des revendications précédentes, pour la ou chaque paire d'électrodes dans le plan comprenant en outre un amplificateur couplé à la paire d'électrodes et ayant une impédance d'entrée supérieure à 1 TΩ et de préférence étant de 200 TΩ.

6. Dispositif de soin capillaire, comprenant :
un capteur de qualité de fibre triboélectrique (S) selon l'une quelconque des revendications précédentes.

7. Dispositif de soin capillaire selon la revendication 6, comprenant en outre un microprocesseur pour analyser le premier signal de tension.

8. Dispositif de soin capillaire selon la revendication 7, comprenant en outre un indicateur pour, en réponse à une sortie du microprocesseur, fournir une indication à un utilisateur que l'utilisateur doit arrêter un traitement afin d'éviter une surchauffe.

9. Dispositif de soin capillaire selon la revendication 7 ou 8, dans lequel le dispositif de soin capillaire est agencé pour modifier un réglage de température en réponse à une sortie du microprocesseur.
